# EUROPEAN PATENT SPECIFICATION

(11) **EP 0 742 712 B1**
(45) Date of publication and mention of the grant of the patent: **01.08.2001**
(21) Application number: 95908234.8
(22) Date of filing: 31.01.1995
(51) Int. Cl.: A61K 9/20, A61K 31/43

(54) **BILAYERED AMOXYCILLIN TABLETS**
AMOXICILLIN ZWEISCHICHTTABLETTEN
COMPRIMES DE TYPE BI-COUCHE A BASE D'AMOXYCILLINE

(30) Priority: 04.02.1994 GB 9402203
(43) Date of publication of application: 20.11.1996
(60) Divisional application: 99204294.5
(73) Proprietor: SMITHKLINE BEECHAM PLC, Brentford, Middlesex TW8 9EP (GB)
(72) Inventor: EBBERS, Walter F.W., CH-4132 Muttenz (CH); ZIMMER, Robert H., CH-4132 Muttenz (CH)
(74) Representative: Connell, Anthony Christopher
(86) International application number: EP9500343
(87) International publication number: WO9520946

(56) References cited:
- WO-A-94/06416
- WO-A-94/27557

## Description

This invention relates to novel tablet formulations for oral administration of amoxycillin together with clavulanic acid.

Amoxycillin and clavulanic acid are respectively a known β-lactam antibiotic and a known β-lactamase inhibitor. Their use together in antibiotic formulations is disclosed in for example GB 2005538, which discloses formulations comprising amoxycillin trihydrate and potassium clavulanate. In oral formulations amoxycillin is generally used in the form of its trihydrate, and clavulanic acid is generally used in the form of a pharmaceutically acceptable salt (hereinafter "clavulanate") in particular potassium clavulanate.

It is desirable to provide oral pharmaceutical formulations which release their active material content at a controlled rate after oral administration, so that for example release of the active material into the stomach or intestine occurs over a period of several hours after ingestion of the formulation. This can allow unit doses of the formulation to be taken at widely separated intervals, for example twice daily, whilst maintaining a therapeutically effective plasma level of the active material content, such as amoxycillin and clavulanate.

Among various ways of achieving a controlled rate of release are layered tablets. For example GB 1346609 discloses three layer tablets having two drug-containing layers located one on each side of an inert disintegrable intermediate layer. The only drug substance mentioned in GB 1346609 is Mepyrizole. US 4122157 discloses a controlled release nitrofurantoin tablet which comprises two adjacent layers, one being a rapid release layer, the other being a slow release layer. US 4,839,177 discloses controlled release tablets comprising a deposit-core containing an active drug substance, with a support platform applied to this deposit core.

WO 94/06416 (Jagotec AG) describes a bilayer tablets in which the first layer has an immediate or controlled release formulation and the second layer has a slow release formulation, modified by the presence of a further low-permeability barrier-type layer surrounding the second layer or placed between the first and second layers. An example is provided therein of a tablet comprising 500mg of amoxycillin trihydrate, distributed equally between an immediate release layer and a slow release layer, with a barrier layer to further modify release from the slow release layer. This application was published on 31 March 1994, after the priority date of the present application.

Particular problems are encountered with making controlled-release formulations which include clavulanate in combination with antibiotics such as amoxycillin. Amoxycillin trihydrate and clavulanate differ substantially in their solubility in water. Potassium clavulanate is very water soluble and amoxycillin trihydrate is relatively insoluble, so it is difficult to avoid rapid leaching out of potassium clavulanate from formulations containing these two in admixture. Also clavulanate is relatively sensitive to hydrolysis, so degradation of clavulanate may occur on contact with aqueous gastrointestinal contents during the relatively long periods between oral administration of controlled-release formulations.

According to this invention a tablet formulation for oral administration comprises a first layer which includes amoxycillin and/or clavulanate, and a second layer which includes amoxycillin and/or clavulanate, provided that the overall tablet contains amoxycillin and clavulanate, wherein the relative rate of release of amoxycillin and/or clavulanate from the first and second layers differs.

Amoxycillin may suitably be used in the form of amoxycillin trihydrate, and clavulanate may be used in the form of potassium clavulanate. The tablet formulation may contain other antibacterial agents in addition to amoxycillin and clavulanate. Amoxycillin, clavulanate and any such other antibacterial agents which may be present are herein singly and collectively referred to as "active material content" unless otherwise specifically identified.

Tablet formulations of this invention may provide extended plasma levels of their active material content, for example of amoxycillin and clavulanate after ingestion.

When the formulation of the invention contains amoxycillin and clavulanate the overall weight ratio of amoxycillin : clavulanate (expressed as the free acid equivalent) may vary between broad limits, for example between 30 : 1 to 1 : 1. Suitably the ratio may be between 8 : 1 to 1 : 1, for example between 7 : 1 to 1 : 1, typically around 4 : 1, eg between 3.5 : 1 and 4.5 : 1. When both of the layers comprise amoxycillin and clavulanate the amoxycillin : clavulanate ratio in each layer may be the same as the overall tablet ratio or there may be different ratios in each of the layers, making up the ratio in the overall tablet. For example one layer may contain amoxycillin without clavulanate and the other layer may contain amoxycillin plus clavulanate, making up the ratio in the overall tablet. For example one layer may contain clavulanate without amoxycillin and the other layer may contain amoxycillin plus clavulanate, making up the ratio in the overall tablet. For example one layer may contain amoxycillin without clavulanate and the other layer may contain clavulanate without amoxycillin, making up the ratio in the overall tablet.

Suitably the tablet formulations of the invention may contain up to the maximum permitted daily dose of amoxycillin and clavulanate per unit dose. The formulations may for example contain nominally around 875 mg of amoxycillin and around 250 mg of clavulanate. Suitably unit dose tablets of the invention may contain the following nominal weights (mg) of amoxycillin: clavulanate (expressed as free acid equivalent); 875 : 125, 500 : 250, 500 : 125; 250 : 125 and 250 : 62.5.

The differing relative rate of release of active material content from the first and second layers of the tablet may be achieved in various ways.

For example differing rates of release may be achieved by a first layer which is a rapid release layer, ie which releases the bulk of its active material content within a relatively short time, for example within 1 hour, eg within 30 minutes after oral injestion, and a second layer which is a slow release layer, ie which releases the bulk of its active material content during a relatively long period after oral ingestion or is a delayed release layer which releases the bulk of its active material content after a period of delay after oral ingestion, either in the stomach or in the intestine.

Rapid release layers may for example be rapid disintegrating layers having a composition similar to that of known rapid-disintegrating tablets. For example, the composition may comprise principally active material content, binders such as plasdone K29-32 (trade mark), compression aids, fillers or diluents such as mannitol, microcrystalline cellulose, and silicon dioxide, eg Syloid (trade mark), disintegrants, eg Explotab (trade mark), Avicel (trade mark) lubricants such as talc, magnesium stearate etc. Suitably such a rapid release layer may comprise around 30 - 70 % (all percentages given herein are on a weight percentage basis unless otherwise stated) eg 50-60% of active material content, around 10 - 30% of fillers/compression aids, and conventional amounts of disintegrants and lubricants etc.

An alternative type of rapid-release layer may be a swellable layer having a composition which incorporates polymeric materials which swell rapidly and extensively in contact with water or aqueous media, to form a water permeable but relatively large swollen mass. Active material content may be rapidly leached out of this mass.

Slow release layers may have a composition which comprises active material content together with a release retarding material. Suitable release retarding materials include pH sensitive polymers, e.g. the known Eudragit (trade mark) polymers, for example Eudragit L (trade mark), i.e polymers based upon methacrylic acid copolymers, used either alone or with a plasticiser, release-retarding polymers which have a high degree of swelling in contact with water or aqueous media such as the stomach contents, polymeric materials which form a gel on contact with water or aqueous media, and polymeric materials which have both swelling and gelling characteristics in contact with water or aqueous media.

Polymers which have a high degree of swelling include, inter alia, cross-linked sodium carboxymethylcellulose, cross-linked hydroxypropylcellulose, high-molecular weight hydroxypropylmethylcellulose, carboxymethylamide, potassium methacrylatedivinylbenzene co-polymer, polymethylmethacrylate, cross-linked polyvinylpyrrolidone, high-molecular weight polyvinylalcohols etc. Gellable polymers include methylcellulose, carboxymethylcellulose, low-molecular weight hydroxypropylmethylcellulose, low-molecular weight polyvinylalcohols, polyoxyethyleneglycols, non-cross linked polyvinylpyrrolidone etc. Polymers simultaneously possessing swelling and gelling properties include medium-viscosity hydroxypropylmethylcellulose and medium-viscosity polyvinylalcohols.

Examples of such polymers include Methocel K4M (Trade Mark), Methocel E5 (Trade Mark), Methocel E5O (Trade Mark), Methocel E4M (Trade Mark), Methocel K15M (Trade Mark) and Methocel K100M (Trade Mark). An example of a suitable polymer mixture is a mixture of Methocel E5 and K4M, for example 1:1, w:w.

Other known release-retarding polymers which may be incorporated include hydrocolloids such as natural or synthetic gums, cellulose derivatives other than those listed above, proteinaceous substances such as acacia, gum tragacanth, locust bean gum, guar gum, agar, pectin, carageenam, soluble and insoluble alginates, carboxypolymethylene, gelatin, casein, zein, Veegum (trade mark) and the like.

Such a slow release layer may contain polymers which rapidly swell in contact with water or aqueous media so that they form a relatively large swollen mass which is not rapidly discharged from the stomach into the intestine.

Suitably such a slow release layer may contain around 30-70% eg 40 - 60%, of active material content, around 15 - 45% of release-retarding polymers, around 0-30% of fillers/compression aids, conventional quantities of disintegrants and lubricants, and some 5 - 20% of soluble excipients.

Alternative types of slow- or delayed- release layer are those in which the active material content is mixed with, coated with, or embedded in a matrix of a poorly soluble or practically insoluble excipient (e.g. calcium phosphate etc) and/or a hydrophobic excipient (e.g. fats or waxes). Poorly soluble or practically insoluble excipients can yield a very hard tablet which would slowly dissolve or erode from the surface without substantial diffusion of surrounding liquid into the body of the tablet. Hydrophobic excipients similarly hinder moisture ingress and release of active material content occurs through erosion and enzymatic digestion. Examples of such waxes include Compritol (trade mark), for example Compritol HD5 and Compritol 888 (trade marks). Compritol wax remains intact in aqueous solutions at all pH, but is digested by lipase enzymes at pH 5-7. Thus active material content such as clavulanate protected with Compritol wax as described above may be released primarily in the small intestine due to the action of intestinal lipase. Typically when the slow- or delayed- release layer includes such a wax, the wax may suitably comprise 10-80% of the layer, e.g. around 20-40%, e.g. 30 % ± 5% by weight.

Delayed-release layers may use the known properties of enteric polymers to delay release of active material content until the whole or part of the tablet is discharged by the stomach into the intestine after oral ingestion. Enteric polymers are insoluble or only slightly soluble in the stomach contents, but relatively soluble in the higher pH intestinal environment. Individual particles, e.g. granules of active material content may be coated with a layer of or made up with an enteric polymer, and embedded or dispersed within a soluble or disintegrable matrix. Alternatively a delayed release layer may comprise a matrix of enteric polymer within which are dispersed granules comprising active material content, and these granules may themselves be coated with an enteric polymer layer. Such granules may include conventional granulating materials. Examples of suitable enteric polymer materials include the known Eudragit (trade mark) polymers discussed above.

Such a slow-or delayed-release layer may also include fillers/compression aids such as microcrystalline cellulose, eg Avicel (trade mark), desiccants, such as silicon dioxide, eg Syloid (trade mark), disintegrants, eg Explotab (trade mark) and polyvinyl-pyrrolidone, eg polyvidon K3O (trade mark), lubricants such as talc or magnesium stearate, pH-controlling agents such as potassium dihydrogen phosphate or substantially insoluble ion-exchange resins, and soluble excipients such as mannitol or other soluble sugars.

Differing rates of release may also be achieved by having a first layer which is a slow or delayed release layer, and a second layer which is also a slow or delayed release layer. Such first and second layers may for example differ in their composition, so that they comprise different quantities, combinations or types of release-retarding materials and/or soluble excipients etc. Additionally or alternatively such first and second layers may for example differ in the relative amounts of active material content in the first and second layers.

Due to the difference in solubility of amoxycillin and clavulanate, the former may be only slowly dissolved out of a layer, whereas the latter, unless the layer is very impervious or hydrophobic, may be dissolved out relatively rapidly. Therefore if a layer contains both amoxycillin and clavulanate it may be at the same time a slow- or delayed- release amoxycillin layer but a rapid-release clavulanate layer. For example such a layer may comprise amoxycillin trihydrate, potassium clavulanate and the erodable polymer Methocel E5 (trade mark).

In this above-described tablet having two slow or delayed release layers, one or both of the first and second layers may for example be slow release layers comprising active material together with release retarding materials as described above. Alternatively one or both of the first or second layers may be a delayed release layer using enteric polymers as described above.

The tablet formulations of the invention may also include one or more barrier layers, which may be located between the respective first and second layers, and/or on one or more of the outer surfaces of the first and second layers, for example the end faces of the layers of a substantially cylindrical tablet. Such barrier layers may for example be composed of polymers which are either substantially or completely impermeable to water or aqueous media, or are slowly erodable in water or aqueous media or biological liquids and/or which swell in contact with water or aqueous media. Suitably the barrier layer should be such that it retains these characteristics at least until complete or substantially complete transfer of the active material content to the surrounding medium.

Suitable polymers for the barrier layer include acrylates, methacrylates, copolymers of acrylic acid, celluloses and derivatives for the barrier layer such as ethylcelluloses, cellulose acetate propionate, polyethylenes and polyvinyl alcohols etc. Alternatively barrier layers may comprise enteric polymers. Barrier layers comprising polymers which swell in contact with water or aqueous media may swell to such an extent that the swollen layer forms a relatively large swollen mass, the size of which delays its rapid discharge from the stomach into the intestine. The barrier layer may itself contain active material content, for example the barrier layer may be a slow or delayed release layer. Barrier layers may typically have an individual thickness of 2mm to 10 microns.

Suitable polymers for barrier layers which are relatively impermeable to water include the Methocel (trade mark) series of polymers mentioned above, e.g. Methocel K100M, Methocel K15M, Methocel E5 and Methocel E50, used singly or combined, or optionally combined with an Ethocel (trade mark) polymer. Such polymers may suitably be used in combination with a plasticiser such as hydrogenated castor oil. The barrier layer may also include conventional binders, fillers, lubricants and compression acids etc such as Polyvidon K30 (trade mark) magnesium stearate, and silicon dioxide, e.g. Syloid 244 (trade mark)

The tablet formulation of the invention may be wholly or partly covered by a coating layer, which may be a protective layer to prevent ingress of moisture, or damage to the tablet, or may be an enteric coating layer, for example of the known Eudragit (trade mark) polymers described above. The coating layer may itself contain active material content, and may for example by a rapid release layer, which rapidly disintegrates in contact with water or aqueous media to release its active material content for example of amoxycillin, or amoxycillin plus clavulanate combined.

As well as active material content etc, the tablet of the invention may also include a pH modifying agent, such as a pH buffer, which may be contained in either rapid-, slow-, or delayed release layers, or in a coating around all or part of the tablet. A suitable buffer is calcium hydrogen phosphate.

The various first and second layers, barrier layers and coating layers may be combined in various ways to form embodiments of tablet formulations of the present invention, as described below.

One embodiment comprises a tablet having a rapid disintegrating first layer which contains amoxycillin and/or clavulanate as active material, and a slow-release second layer which comprises amoxycillin and/or clavulanate as active material together with one or more release-retarding polymers such as Methocel E5, E50, E4M, K4M, K15M and K100M and mixtures thereof, with a barrier layer of either a substantially completely impermeable polymer or a slowly erodable polymer sandwiched between the first and second layers, the overall tablet containing amoxycillin or amoxycillin plus clavulanate. Suitably the rapid disintegrating and slow release layers may both contain only amoxycillin as active material, or the rapid disintegrating and slow release layers may both contain amoxycillin plus clavulanate, or only the rapid disintegrating layer may contain clavulanate and the slow release layer may contain only amoxycillin as active material content.

Typically the first layer in such a tablet contains around 20-35%, eg around 25-30% of the overall tablet active material content. Suitably the first layer comprises 15-35%, the second layer comprises 50-70%, and the barrier layer comprises 5 - 30% of the overall tablet weight.

A further embodiment comprises a tablet having a rapid-disintegrating first layer which contains amoxycillin and/or clavulanate as active material, and a slow-release second layer which comprises amoxycillin and/or clavulanate as active material together with one or more release retarding polymers such as Methocel E5, E50, E4M, K4M, K15M or K100M or mixtures thereof, but without a barrier layer, the overall tablet containing amoxycillin or amoxycillin plus clavulanate. Suitably the first layer in such a tablet contains some 20 - 35%, eg 25 - 30% of the overall active material content.

A further embodiment comprises a slow-release first layer which contains amoxycillin and/or clavulanate as active material together with one or more release retarding polymers, and a second layer which is also a slow-release layer and which contains amoxycillin and/or clavulanate as active material together with one or more release retarding polymers, and a barrier layer of either a completely impermeable polymer or a slowly erodable polymer sandwiched between the first and second layers, the overall tablet containing amoxycillin or amoxycillin plus clavulanate.

Suitably in this embodiment the first layer may contain amoxycillin without clavulanate and the second layer may contain amoxycillin plus clavulanate, or the first and second layers may both contain amoxycillin plus clavulanate, or the first layer may contain amoxycillin without clavulanate and the second layer may contain clavulanate without amoxycillin. Suitable release-retarding polymers include Methocel E5, E50, E4M, K4M, K15M and K100M or mixtures thereof. Suitably the first and second layers in such a tablet may each contain some 30 - 60% of the active material content making up the overall tablet content of 100%. Suitably the first layer comprises 30 - 60%, the second layer comprises 30-60% and the barrier layer comprises 5-20%, of the overall tablet weight.

A further embodiment comprises a slow-release first layer which comprises amoxycillin and/or clavulanate as active material together with one or more release retarding polymers, and a slow-release second layer which comprises amoxycillin and/or clavulanate as active material together with one or more release retarding polymers, and a barrier layer of either a substantially completely impermeable polymer or a slowly erodable polymer located on one of the end faces of the tablet.

Suitably the first layer may contain amoxycillin without clavulanate as its active material and the second layer may contain amoxycillin plus clavulanate (eg in a 1 : 1 ratio) as its active material, with the barrier layer located on the end face of the second layer, the overall tablet containing amoxycillin or amoxycillin plus clavulanate. Suitable release-retarding polymers are Methocel E5, E50, E4M, K4M, K15M and K100M or mixtures thereof. Suitably the first layer and second layers may respectively contain some 35 - 70% and 20 - 50% of the active material content. Suitably the first layer comprises 35 - 70%, the second layer 20 - 50% and the barrier layer 5 - 20% of the overall tablet weight.

A further embodiment comprises a rapid release first layer which is a swellable layer as described above containing amoxycillin, and a slow-release or delayed release second layer as described above containing amoxycillin, or clavulanate, or amoxycillin plus clavulanate, having a barrier layer of either a substantially or completely impermeable polymer or a slowly erodable polymer sandwiched between the first and second layers.

A further embodiment comprises a slow-release first layer comprising amoxycillin optionally together with clavulanate as active material, and a second layer which is a delayed release layer comprising a disintegrable or soluble matrix within which are dispersed enteric polymer coated granules which comprise clavulanate, optionally together with amoxycillin, with a barrier layer sandwiched between the first and second layers. Suitably the first layer may comprise amoxycillin and a Methocel (trade mark) polymer such as Methocel E5, and the second layer may comprise granules of a mixture of amoxycillin and clavulanate, together with a suitable granulating material such or a Methocel (trade mark) polymer such as Methocel K4M, coated with a Eudragit polymer such as Eudragit L.

A further embodiment comprises a first layer which is a slow release layer containing polymers which rapidly swell in contact with water or aqueous media so that the swollen mass is not rapidly discharged from the stomach containing amoxycillin or amoxycillin plus clavulanate as active material, and a second layer which is a slow release layer containing amoxycillin or amoxycillin plus clavulanate as active material, with a barrier layer of either a completely impermeable polymer or a slowly erodable polymer sandwiched between the first and second layers. The tablet of this embodiment may optionally be surrounded by a coating layer which is a rapidly disintegrating composition containing amoxycillin and clavulanate as active material.

A further embodiment is a tablet comprising three layers tablet in which all the three layers include an active material content. Suitably such a tablet may comprise a slow-or delayed-release first layer which comprises amoxycillin, optionally together with clavulanate, a rapid-release second layer which comprises amoxycillin, optionally together with clavulanate, and a third layer, sandwiched between the first and second layers, and being a slow- or delayed-release layer comprising calvulanate, optionally together with amoxycillin. Suitably in such a tablet the first layer may include amoxycillin without clavulanate, the second layer may comprise a mixture of amoxycillin plus clavulanate, and the third layer may comprise clavulanate or a mixture of clavulanate and amoxycillin. The first layer may suitably comprise amoxycillin and a release-retarding polymer such as Methocel E5 (trade mark) optionally together with a swellable material, the second layer may comprise a mixture of amoxycillin and clavulanate together with conventional fillers, binders, disintegrants etc, and the third, intermediate layer may comprise amoxycillin, clavulanate and a release retarding polymer such as Methocel K100M.

Each of the above-mentioned embodiments of the invention may be coated with a polymer layer, for example an enteric polymer such as the Eudragit (trade mark) polymers mentioned above, or a coating layer which contains active material content, such as a rapid-release layer which includes amoxycillin and/or clavulanate.

Suitably the tablet formulations of the invention may be formed by known compression tabletting techniques for example using a known multi-layer tabletting press. Suitably a dry densification process may be used, e.g. briquetting. Typically the active material content, pH modifiers, buffers, fillers and/or diluent, release retarding agents, disintegrants and binders, when used are mixed, then lubricants and compression aids are added. The complete mixture may then be compressed under high pressure in the tablet press. Roller compaction may be used to form granulates for compaction. Alternatively wet granulation may be used, isopropanol being a suitable solvent. A suitable wet granulating aid is Polyvidon K-30 (trade mark).

For making up layers which include a wax, e.g. Compritol (trade mark) dry compression or wet granulation may be used. Typically for wet granulation a colloidal solution or suspension of the wax in a solvent such as dichloromethane may be used. Typically the solution or suspension may be mixed with the layer ingredients and the wet cake sieved and dried. This can be repeated until the required amount of wax has been incorporated.

The barrier layer may typically be made up by a wet granulation technique, or by dry granulation techniques such as roller compaction. Typically the barrier material, e.g. Methocel (trade mark) is suspended in a solvent such as ethanol containing a granulation acid such as Ethocel or Polyvidon K-30 (trade mark), followed by mixing, sieving and granulation. Typically a first layer may be formed, then a barrier layer deposited upon it, eg by compression, spraying or immersion techniques, then the second layer may be formed so that the barrier layer is sandwiched between the first and second layers. Additionally or alternatively the first and second layers may be formed and a barrier layer may then be formed, eg by compression, spraying or immersion, on one or more of the end faces of the tablet. An enteric polymer coating may be applied to the whole or part of the tablet formulation of the invention by conventional spraying or immersion techniques.

For applying an enteric coating to particles used to make up the layers of the tablet of the invention, typically a solution or suspension of the enteric polymer, e.g. Eudragit (Trade Mark), is mixed with the active material content, e.g. powders or granules e.g. make up as described above, and the wet cake is sieved and dried. This procedure may be repeated until the required amount of the polymer is incorporated. Alternatively the granule ingredients or the granules themselves may be coated with enteric polymer by means of a fluid bed granulator, for example spray-coating the ingredients with a solution of the polymer e.g. in isopropanol.

Clavulanic acid and its derivatives are known to be extremely water sensitive, potassium clavulanate being the most stable pharmaceutically acceptable derivative. Therefore formulations which contain derivatives of clavulanic acid, such as potassium clavulanate, should be made up in dry conditions, preferably at 30% relative humidity or less, and the ingredients of the formulation should be predried where appropriate. Formulations of the invention which include derivatives of clavulanic acid should be stored in containers which are sealed against the ingress of atmospheric moisture.

The invention also provides a method for the manufacture of a tablet formulation as described above comprising the steps of forming said first and second layers, and any barrier layers and coating layer(s) which may be present.

The invention further provides a formulation as described above for use as a therapeutic substance for oral administration for the treatment of bacterial infections.

The invention further provides a method of use of a formulation as described above, in the manufacture of a medicament for the treatment of bacterial infections.

The invention further provides a method of treatment of bacterial infections in humans or animals which comprises the administration thereto of a therapeutically effective amount of active material content included in a formulation as described herein.

The invention will now be described by way of example only with reference to the accompanying drawings, in which:

Fig. 1 shows the structure of various types of tablet formulation of the present invention.

Figs. 2, 3, 4, 5 and 6 show *in vitro* and *in vivo* release characteristics from tablet 6-1.

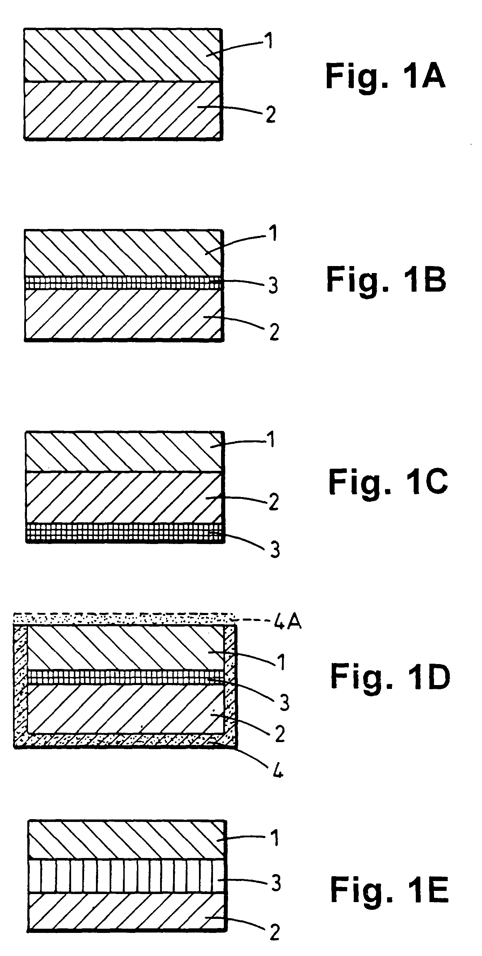

Referring to Fig 1, substantially cylindrical compressed tablets of the present invention are shown in longitudinal section. In Fig 1A, the tablet comprises a first layer (1) and a second layer (2), without any barrier layer or coating layer. In Fig 1B the tablet comprises a first layer (1), a second layer (2), and a barrier layer (3) sandwiched between the first and second layers (1) and (2). In Fig 1C, the tablet comprises a first layer (1), a second layer (2), and a barrier layer (3) located on the end face of the second layer (2). In Fig 1D the tablet comprises a first layer (1), a second layer (2), a barrier layer (3) sandwiched between the first and second layers (1) and (2), and a coating layer (4) which partly covers the tablet. The dotted line shows the possibility of the coating layer (4A) covering the entire tablet. In Fig. 1E the tablet comprises a first layer (1) a second layer (2), and a third layer (3) intermediate between the first and second layers (1) and (2). All three of these layers (1), (2) and (3) include active material content.

(In the examples and figures below "AMX" is an abbreviation for amoxycillin, and "KCA" is an abbreviation for potassium clavulanate).

### Barrier Layers

In the Examples below, barrier layers L1, S2, S6 and RSB1 are referred to. The composition of these layers and the method for preparing them was as follows:

| | Amounts in % | | | |
|---|---|---|---|---|
| | **L1** | **S2** | **S6** | **RSB1** |
| Methocel K100M | 79.75% | -- | -- | -- |
| Methocel K15M | -- | -- | -- | 38.20% |
| Methocel E5 | -- | 75.50% | -- | -- |
| Methocel E50 | -- | -- | 76.50 | -- |
| Mannitol | -- | -- | -- | 38.20% |
| Hydrogenated castor oil | 13.50% | 18.80% | 18.40% | 18.50% |
| Yellow FCF aluminium lake | 0.25% | -- | -- | -- |
| Eudralak green | -- | 0.10% | 0.10% | 0.10% |
| Ethocel | 5.00% | -- | -- | -- |
| Polyvidon K30 | -- | 2.80% | 3.50% | 3.50% |
| Mg-Stearate | 1.00% | 1.90% | 1.00% | 1.00% |
| Syloid 244 | 0.50% | 0.90% | 0.50% | 0.50% |
| Total: | 100.00% | 100.00% | 100.00% | 100.00% |

The binding ingredient (Ethocel or Polyvidon K-30) was dissolved in Ethanol (94%). Methocel, Cutina HR and dye were mixed for 5 minutes in a Lödige MGT 30P at 150 r.p.m. after which the granulation solution was added. Mixing was continued for another 5 minutes. The wet granulate was dried at 45°C. The dry granulate was passed through a 1 mm sieve. Mg-stearate and Syloid 244, which had been passed through a 0.71 mm sieve, were added and the whole was mixed in the Lödige during 1 min at 150 r.p.m. to homogeneity

### Preparation of Tablets

The active ingredients, fillers and diluents (mannitol), release controlling agents (Methocels) or disintegrants (Avicel, Explotab) and binders (Plasdone K29-32) were mixed during 5 minutes in a Stefan Mixer at speed level 11. Lubricants (talc, Mg-stearate) and colloidal silicon dioxide (Syloid 244) were added, and mixing at level 1 was continued for another minute. The complete mixture was slugged on a Kilian tablet press (briquetting step), followed by size reduction (Frewitt) and passage through an oscillatory 1 mm sieve. If the flow properties were unsatisfactory, the briquetting step was repeated. The tablets of Examples 1 to 4 were prepared in this way.

The tablets of subsequent Examples were prepared by non-aqueous wet granulation. In most cases, isopropanol was chosen as the most appropriate non-aqueous solvent. In the formulations with Compritol, dichloromethane was used. In the non-aqueous granulation step an isopropanolic solution of polyvidon K-30 was mixed with a powder mixture of active ingredients and methocels (and fillers, buffers, etc.). The wet cake was pressed through a 0.5 mm sieve, the mixing was repeated and followed by sieving through a 2 mm sieve. After drying at 40°C the dry material was passed through a 1 mm sieve. Magnesium stearate and talc were added and mixed.

This granulate possessed good flow properties. In some cases, where the bulk density was rather low a densifying step (pre-tabletting and sieving as in the briquetting method) was still required in order to achieve the nominal weight of a particular layer.

Coating particles and granules with Eudragit L was performed in a similar manner. A solution of Eudragit in isopropanol was mixed with the active ingredients or with a granulate (without talc and Mg-stearate) prepared in the aforementioned wet granulation manner. The wet cake was pressed through a 0.5 mm sieve, the mixing was repeated and followed by sieving though a 2 mm sieve. After drying at 40°C the dry material was passed through a 1 mm sieve. The treatment with Eudragit, sieving and drying was repeated until the nominal amount of Eudragit L according to the recipe was contained within the granulate. Then Mg-stearate and talc were added and mixed.

Other coating work with Eudragit L involved the use of a fluid bed granulator. The active ingredients were spray-coated with an isopropanolic solution of Eudragit L in a Glatt fluid bed granulator to 20% Eudragit L of the weight of the active ingredients. Samples were taken at the 5, 10, 15 and 20% Eudragit weight level. In addition, a granulate of active ingredients with Methocels was prepared in the Glatt employing an isopropanolic solution of polyvidon K-30. This granulate was then spray-coated with Eudragit L on the Glatt.

The formulation work with Compritol either involved dry compression or a wet granulation step with a colloidal solution of Compritol in dichloromethane. The wet granulation was analogous to the Eudragit wet granulation. The treatment with the Compritol solution, sieving and drying was repeated until the nominal amount of Compritol according to the recipe was contained within the granulate. Then Mg-stearate and talc were added and mixed.

### Dissolution Testing Methods

The release of amoxycillin and clavulanate from tablets into static media was measured using the USP Dissolution Test Method II.

The release of amoxycillin and clavulanate from tablets into flowing water and other media was measured using the USP Method IV Dissolution , using large (22.6 mm, 8.3 ml/min) and small (12 mm 8.3 ml/min) cells.

The test was performed according to USP XXXII <724>, apparatus 4.

| **Dissolution apparatus** | |
|---|---|
| Dissolution apparatus | SOTAX CE6 flow cell |
| | SOTAX CY7-50 pump |

| **Test specifications** | |
|---|---|
| Temperature | 37.0 ± 0.5 °C |
| Test medium | 0 - 2h: HCl 0.01 M (pH 2.0) |
| | 2 - 8h: phosphate buffer 0,05 M; pH = 6.6 |
| Flow rate | 8.3 ml/min (500 ml/h) |
| Flow cell | 22.6 mm diameter |

### Solutions:

- 0.05 M phosphate buffer solution 3 (pH 6.6): Dissolve 544g potassium dihydrogenphosphate in 20.0L water and adjust to pH 6.6 with NaOH 40% (m/v).
- 0.05 M phosphate buffer solution 4 (pH 6.6): Dissolve 408g potassium dihydrogenphosphate and 39g sodium hydroxide pellets p.a. in 60L water.

### Method:

Three tablets were tested each as follows. A 5 mm diameter ruby bead was placed at the bottom of the cone of the flow cell and the cone was filled with 1 mm diameter glass beads. A tablet was introduced into the cell and fixed vertically using a holder. The filter head was assembled and the parts fixed together by means of a clamping device. The dissolution medium warmed to 37.0 ± 0.5°C was introduced through the bottom of the cell at a flow rate of 8.3 ml/min. 0.01 M HCl from the beginning to 2 hours was used, then changed to buffer solution 4 until the end of the run.

The drug release after 30 min, 60 min 2 h 3 h, 4 h, 5 h, 6 h, 7 h and 8 h was determined.

As Potassium Clavulanate is very unstable at low pH values, the fractions for the time from 0 to 2 hours (medium 0.01 M HCL) were collected directly in a buffer solution and were mixed well with this buffer in a ratio of about 1:1.

### Example 1

Tablets 1.1, 1.2 and 1.3, each having a structure as shown in Fig 1B were prepared using an oval biconvex punch 21 x 10 mm, having a rapid-disintegrating first layer and a slow-release second layer, with a barrier layer between. The composition of these tablets is as follows:

| | Amounts in mg | | |
|---|---|---|---|
| | **1.1** | **1.2** | **1.3** |
| **First Layer** | | | |
| AMX.3H₂O | 160.7 | 160.7 | 160.7 |
| (= AMX f.a.) | (138.8) | (138.8) | (138.8) |
| Avicel PH 102 | 44.8 | 44.8 | 44.8 |
| Explotab | 11.2 | 11.2 | 11.2 |
| Talc | 22.3 | 22.3 | 22.3 |
| Mg-Stearate | 8.4 | 8.4 | 8.4 |
| Syloid 244 | 5.6 | 5.6 | 5.6 |
| weight of layer | 253.0 | 253.0 | 253.0 |
| | | | |

| **Barrier Layer** | L1 | L1 | S2 |
|---|---|---|---|
| weight of layer | 180.0 | 180.0 | 180.0 |
| | | | |

| **Second Layer** | | | |
|---|---|---|---|
| AMX.3H₂O | 413.3 | 413.3 | 413.3 |
| (= AMX f.a.) | (357.1) | (357.1) | (357.1) |
| Methocel K4M | 72.0 | 72.0 | 72.0 |
| Methocel E5 | 72.0 | 72.0 | 72.0 |
| Mannitol | 72.0 | 26.0 | 72.0 |
| KH₂PO₄ | - | 46.0 | - |
| Polyvidon K30 | 28.6 | 28.6 | 28.6 |
| Talc | 7.2 | 7.2 | 7.2 |
| Mg-Stearate | 2.9 | 2.9 | 2.9 |
| | | | |
| weight of layer | 668.0 | 668.0 | 668.0 |
| **Total tablet weight** | **1101.0** | **1101.0** | **1101.0** |
| Total content AMX f.a. | 495.9 | 495.9 | 495.9 |

### Example 2

Tablets 2.1 and 2.2 each having a structure as shown in Fig. 1B were prepared using an oval biconvex punch 21 x 10mm, having a first layer which is a rapid disintegrating layer, a second layer which is a slow-release layer, and a barrier layer sandwiched between them. These tablets had the following composition:

| | Amounts in mg | |
|---|---|---|
| **First Layer** | **2.1** | **2.2** |
| AMX.3H₂O | 118.7 | 118.7 |
| (= AMX f.a.) | (102.6) | (102.6) |
| AMX.3H₂O : KCA | 83.4 | 83.4 |
| 1:1* | | |
| (= AMX f.a.) | (35.1) | (35.1) |
| (= CA f.a.) | (36.3) | (36.3) |
| Avicel PH 102 | 45.1 | 45.1 |
| Explotab | 11.4 | 11.4 |
| Talc | 22.4 | 22.4 |
| Mg-Stearate | 8.4 | 8.4 |
| Syloid 244 | 5.6 | 5.6 |
| | | |
| weight of layer | 295.0 | 295.0 |
| | | |

| **Barrier Layer** | L1 | L1 |
|---|---|---|
| weight of layer | 160.0 | 160.0 |
| | | |

| **Second Layer** | | |
|---|---|---|
| AMX.3H₂O | 305.27 | 305.27 |
| (= AMX f.a.) | (263.75) | (263.75) |
| AMX.3H₂O : KCA | 214.40 | 214.40 |
| 1:1* | | |
| (= AMX f.a.) | (90.26) | (90.26) |
| (= CA f.a.) | (93.26) | (93.26) |
| Methocel K4M | 62.00 | 62.00 |
| Methocel E5 | 62.00 | 62.00 |
| Mannitol | 46.00 | - |
| KH₂PO₄ | - | 46.00 |
| Polyvidon K30 | 10.03 | 10.03 |
| Talc | 5.00 | 5.00 |
| Mg-Stearate | 20.30 | 20.30 |
| weight of layer | 725.00 | 725.00 |
| **Total tablet weight** | **1180.00** | **1180.00** |
| | | |
| Total content AMX f.a. | 491.7 | 491.7 |
| Total content CA f.a. | 129.6 | 129.6 |

| | | |
|---|---|---|
| * **Note:** Potassium clavulanate was used in the form of a commercially available 1 : 1 mixture of potassium clavulanate and amoxycillin trihydrate. | | |

### Example 3

Tablets 3.1 and 3.2 each having a structure as shown in Fig 1C were prepared, using respectively oval biconvex punch 18 x 8 mm and 21 x 10 mm, having a first layer which is a slow release layer, a second layer which is also a slow release layer and a barrier layer located on the end face of the second layer. These tablets had the following composition:

| | Amounts in mg | |
|---|---|---|
| **First Layer** | **3.1** | **3.2** |
| AMX.3H₂O | 305.27 | 327.52 |
| (= AMX f.a.) | (263.75) | (282.98) |
| Methocel K4M | 53.18 | 57.06 |
| Methocel E5 | 53.18 | 57.06 |
| Mannitol | 53.18 | 57.06 |
| Polyvidon K30 | 18.03 | 19.34 |
| Talc | 5.31 | 5.70 |
| Mg-Stearate | 5.85 | 6.26 |
| | | |
| weight of layer | 494.00 | 530.00 |
| | | |

| **Second Layer** | | |
|---|---|---|
| AMX.3H₂O : KCA 1:1 | 202.04 | 214.40 |
| (= AMX f.a.) | (85.06) | (90.26) |
| (= CA f.a.) | (87.89) | (93.26) |
| Methocel K4M | 35.20 | 37.35 |
| Methocel E5 | 35.20 | 37.35 |
| Mannitol | 35.20 | 37.35 |
| Polyvidon K30 | 11.99 | 12.72 |
| Talc | 3.52 | 3.73 |
| Mg-Stearate | 3.85 | 4.10 |
| | | |
| weight of layer | 327.00 | 347.00 |
| | | |

| **Barrier Layer** | **S6** | **S6** |
|---|---|---|
| weight of layer | 150.00 | 180.00 |
| | | |
| **Total tablet weight** | **971.00** | **1057.00** |
| | | |
| Total content AMX f.a. | 348.8 | 373.3 |
| Total content CA f.a. | 87.9 | 93.3 |

### Example 4

Tablets 4.1, 4.2, 4.3, 4.4, 4.5 and 4.6, each having a structure as shown in Fig 1B were prepared, using oval biconvex punches 21 x 10 mm, having a first layer which is a slow-release layer, a second layer which is also a slow-release layer, and a barrier layer sandwiched between the first and second layers. These tablets had the following compositions.

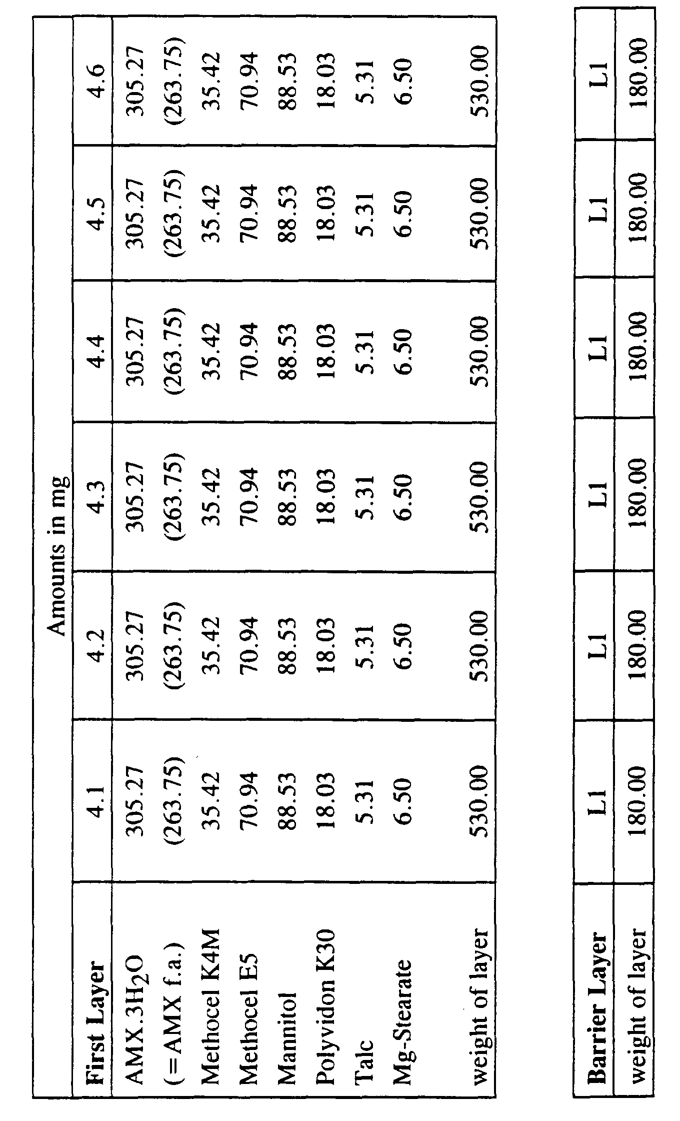

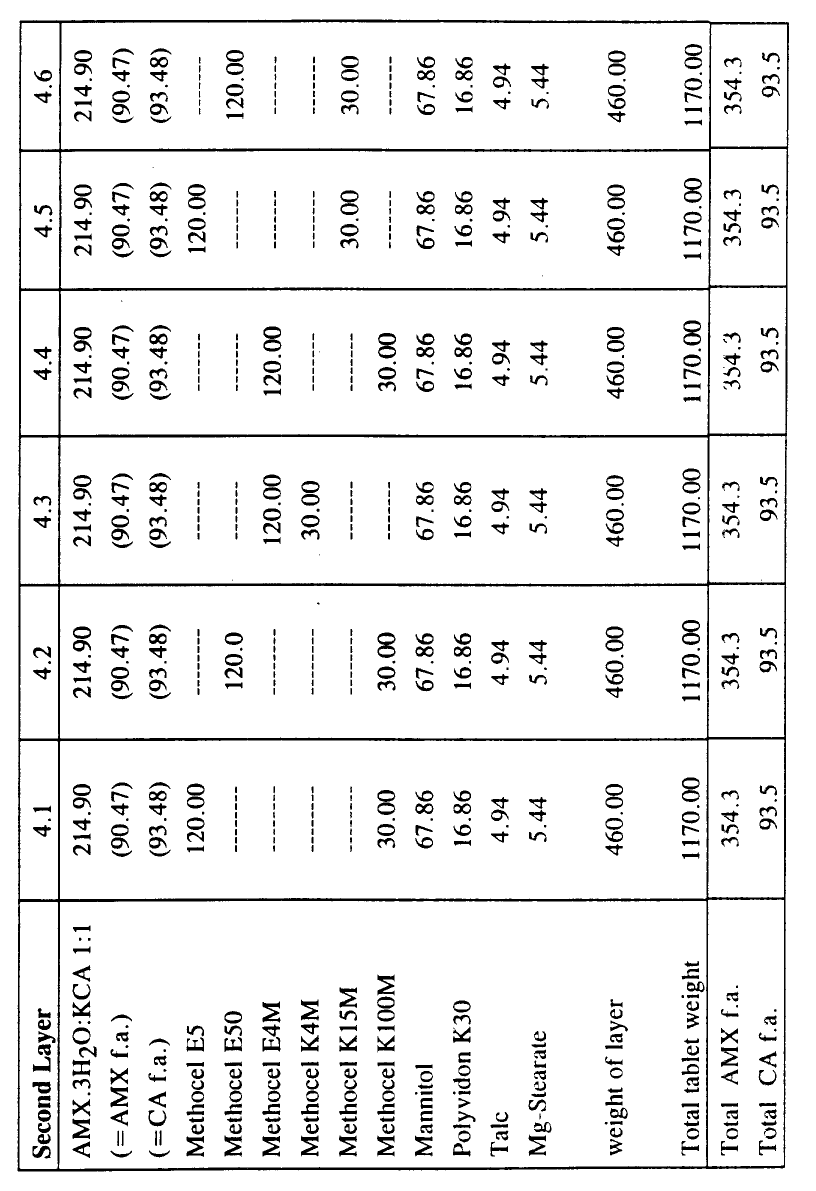

### Example 5

Tablets 5.1, 5.2, 5.3, and 5.4 were prepared having a structure as shown in Fig. 1B.

| Amounts | | | | |
|---|---|---|---|---|
| **First Layer** | 5.1 | 5.2 | 5.3 | 5.4 |
| AMX.3H₂O(mg) | 320 | 215 | 320 | 210 |
| Methocel K4M% | 8.2 | 8.2 | 8.2 | 8.2 |
| Methocel E5% | 16.4 | 16.4 | 16.4 | 16.4 |

| **Barrier Layer** | L1 | L1 | L1 | L1 |
|---|---|---|---|---|
| **Second Layer** | | | | |
| AMX.3H₂O (mg) | 121 | 121 | 121 | 121 |
| KCA (mg) | 125 | 125 | 125 | 125 |
| Methocel K4M % | 10 | 30 | ------- | ------- |
| Methocel K100M % | ----- | ----- | 10 | 20 |

### Example 6

Tablets 6.1 and 6.2 were prepared having a structure as shown in Fig. 1C.

| | Amounts in mg | |
|---|---|---|
| **First Layer** | **6.1** | **6.2** |
| AMX.3H₂O | 438.71 | 438.71 |
| (= AMX f.a.) | (379.05) | (379.50) |
| AMX.3H₂O/K-CA 1:1 | 103.45 | 103.45 |
| (= AMX f.a.) | (43.55) | (43.55) |
| (= CA f.a.) | (45.0) | (45.0) |
| Methocel K4M | 26.91 | 26.91 |
| Methocel E5 | 107.63 | 107.63 |
| Polyvidon K30 | 25.20 | 25.20 |
| Talc | 9.36 | 9.36 |
| Mg-Stearate | 8.64 | 8.64 |
| weight of layer 1 | 719.90 | 719.90 |

| **Second Layer** | | |
|---|---|---|
| AMX.3H₂O/K-CA 1:1 | 183.91 | 183.91 |
| (= AMX f.a.) | (77.4) | (77.4) |
| (CA f.a.) | (80.0) | (80.0) |
| Methocel K15M | 32.04 | ------- |
| Methocel K100M | ----- | 32.04 |
| Methocel E50 | 32.04 | 32.04 |
| Polyvidon K30 | 8.75 | 8.75 |
| Talc | 3.30 | 3.30 |
| Mg-Stearate | 3.05 | 3.05 |
| | | |
| weight of layer 2 | 263.09 | 263.09 |

| **Barrier layer** | S6 | S6 |
|---|---|---|
| **weight of layer** | 180.00 | 180.00 |
| | | |
| **Total tablet weight** | **1162.99** | **1162.99** |

### Example 7

Tablets 7.1, 7.2 and 7.3 were prepared having a structure as shown in Figs 1B (7.1) and 1E (7.2, 7.3).

| | Amounts in mg |
|---|---|
| **First Layer** | **7.1** |
| AMX.3H₂O | 438.71 |
| (= AMX f.a.) | (379.05) |
| AMX.3H₂O/K-CA 1:1 | 103.45 |
| (= AMX f.a.) | (43.55) |
| (= CA f.a.) | (45.00) |
| Methocel E5 | 134.64 |
| Polyvidon K30 | 25.20 |
| Talc | 9.36 |
| Mg-Stearate | 8.64 |
| weight of layer 1 | 720.00 |

| **Barrier layer** | L1 |
|---|---|
| **weight of layer** | 180.00 |

| **Second Layer** | |
|---|---|
| AMX.3H₂O/K-CA 1:1 | 183.91 |
| (= AMX f.a.) | (77.4) |
| (= CA f.a.) | (80.0) |
| Methocel K100M | 49.71 |
| Polyvidon K30 | 8.70 |
| Talc | 3.23 |
| Mg-Stearate | 2.98 |
| | |
| weight of layer 3 | 248.53 |
| **Total tablet weight** | **1148.53** |

In tablet 7.1 the potassium clavulanate in the first layer is a rapid release component, as the erodable polymer Methocel E5 allows rapid dissolution of soluble potassium clavulanate.

| | Amounts in mg | |
|---|---|---|
| **First Layer** | **7.2** | **7.3** |
| AMX.3H₂O | 319.96 | 319.96 |
| (= AMX f.a.) | (276.45) | (276.45) |
| Methocel E5 | 100.00 | 100.00 |
| Mannitol | 50.04 | 50.04 |
| Polyvidon K30 | 17.50 | 17.50 |
| Talc | 6.50 | 6.50 |
| Mg-Stearate | 6.00 | 6.00 |
| | | |
| weight of layer 1 | 500.00 | 500.00 |

| **Third Layer** | | |
|---|---|---|
| AMX.3H₂O/K-CA 1:1 | 203.91 | 203.91 |
| (= AMX f.a.) | (85.85) | (85.85) |
| (= CA f.a.) | (88.70) | (88.70) |
| Methocel K100M | 55.12 | 80.01 |
| CaHPO₄ | ----- | 92.08 |
| Polyvidon K30 | 9.65 | 14.01 |
| Talc | 3.58 | 5.20 |
| Mg-Stearate | 3.30 | 4.79 |
| weight of layer 2 | 275.56 | 400.00 |

| **Second Layer** | | |
|---|---|---|
| AMX.3H₂O | 118.7 | 118.7 |
| (= AMX f.a.) | (102.6) | (102.6) |
| AMX.3H₂O/K-CA 1:1 | 83.4 | 83.4 |
| (= AMX f.a.) | (35.1) | (35.1) |
| (= CA f.a.) | (36.3) | (36.3) |
| Avicel PH 102 | 45.1 | 45.1 |
| Explotab | 11.4 | 11.4 |
| Polyvidon K30 | 10.0 | 10.0 |
| Talc | 12.4 | 12.4 |
| Mg-Stearate | 8.4 | 8.4 |
| Syloid 244 | 5.6 | 5.6 |
| | | |
| weight of layer 3 | 295.0 | 295.0 |
| **Total tablet weight** | 1070.56 | 1195.00 |

In tablets 7.2 and 7.3 all three of the layers contain active material content, first and second layers being slow-release layers, and the intermediate third layer being a rapid-release layer.

### Example 8

Tablets 8.1 and 8.2 were prepared having a structure as shown in Fig. 1B.

| | Amounts in mg | |
|---|---|---|
| **First Layer** | **8.1** | **8.2** |
| AMX.3H₂O | 118.7 | 118.7 |
| (= AMX f.a.) | (102.6) | (102.6) |
| AMX.3H₂O/K-CA 1:1 | 83.4 | 83.4 |
| (= AMX f.a.) | (35.1) | (35.1) |
| (= CA f.a.) | (36.3) | (36.3) |
| Avicel PH 102 | 45.1 | 45.1 |
| Explotab | 11.4 | 11.4 |
| Polyvidon K30 | 10.0 | 10.0 |
| Talc | 12.4 | 12.4 |
| Mg-Stearate | 8.4 | 8.4 |
| Syloid 244 | 5.6 | 5.6 |
| weight of first layer | 295.0 | 295.0 |

| **Barrier layer** | S6 | RSB1 |
|---|---|---|
| **weight of layer** | 180.00 | 180.00 |

| **Second Layer** | | |
|---|---|---|
| AMX.3H₂O | 320.02 | 320.02 |
| (= AMX f.a) | (276.5) | (276.5) |
| AMX.3H₂O/K-CA 1:1 | (203.91) | 203.91 |
| (= AMX f.a.) | (85.8) | (85.8) |
| (=CA f.a.) | (88.7) | (88.7) |
| Methocel K4M | 62.00 | 62.00 |
| Methocel E5 | 62.00 | 62.00 |
| Mannitol | 31.74 | 31.74 |
| Polyvidon K30 | 20.03 | 20.03 |
| Talc | 5.00 | 5.00 |
| Mg-Stearate | 20.30 | 20.30 |
| | | |
| weight of second layer | 725.00 | 725.00 |
| Total tablet weight | 1200.00 | 1200.00 |

### Example 9

Tablets 9.1 and 9.2 were prepared having a structure as shown in Figs 1B (9.1) and 1C (9.2).

| | Amounts in mg | |
|---|---|---|
| **First Layer** | **9.1** | **9.2** |
| AMX.3H₂O | 329.05 | 329.05 |
| (= AMX f.a.) | (248.3) | (248.3) |
| Methocel E5 | 102.84 | 102.84 |
| Mannitol | 51.46 | 51.46 |
| Polyvidon K30 | 18.00 | 18.00 |
| Talc | 6.68 | 6.68 |
| Mg-Stearate | 6.17 | 6.17 |
| | | |
| weight of first layer | 514.20 | 514.20 |

| | **9.1** | **9.2** |
|---|---|---|
| **Barrier layer** | L1 | Second layer of 9.1 |
| **weight of layer 2** | 180.00 | 374.82 |

| **Second Layer** | **9.1** | **9.2** |
|---|---|---|
| AMX.3H₂O/K-CA 1:1 | 215.52 | Barrier L1 |
| (= AMX f.a.) | (90.73) | |
| (= CA f.a.) | (93.75) | |
| Methocel K4M | 74.96 | |
| Eudragit L* | 56.22 | |
| Dibutylphthalate* | 5.62 | |
| Polyvidon K30 | 13.12 | |
| Talc | 4.87 | |
| Mg-Stearate | 4.50 | |
| | | |
| weight of layer3 | 374.81 | |
| Total tablet weight | 1069.01 | 1069.01 |

| | | |
|---|---|---|
| * In these tablets the Eudragit L and plasticiser were coated directly onto the active material content by repeated steps of wet granulation. Two further examples of tablets (9.3 and 9.4) were prepared to the formula for 9.1 and 9.2 by first preparing a granulate of amoxycillin plus potassium clavulanate plus Methocel K4M, then coating the granulate with the Eudragit L. | | |

### Example 10

Tablets 10.1 and 10.2 having a structure as shown in Fig. 1C were prepared

| | Amounts in mg | |
|---|---|---|
| **First Layer** | **10.1** | **10.2** |
| AMX.3H₂O | 438.68 | 438.68 |
| (= AMX f.a.) | (379.0) | (379.0) |
| Methocel E5 | 53.00 | 53.00 |
| Mannitol | 6.52 | 6.52 |
| Polyvidon K30 | 18.55 | 18.55 |
| Talc | 6.89 | 6.89 |
| Mg-Stearate | 6.36 | 6.36 |
| | | |
| weight of first layer 1 | 530.00 | 530.00 |

| **Second Layer** | | |
|---|---|---|
| AMX.3H₂O/K-CA 1:1 | 287.36 | 287.36 |
| (= AMX f.a.) | (121.0) | (121.0) |
| (= CA f.a.) | (125.0) | (125.0) |
| Methocel K4M | 34.50 | ------- |
| Methocel K100M | ----- | 34.50 |
| Mannitol | 2.44 | 2.44 |
| Polyvidon K30 | 12.08 | 12.08 |
| Talc | 4.48 | 4.48 |
| Mg-Stearate | 4.14 | 4.14 |
| | | |
| weight of layer 2 | 345.00 | 345.00 |

| **Barrier layer** | S6 | S6 |
|---|---|---|
| **weight of layer 2** | 180.00 | 180.00 |
| | | |
| **Total tablet weight** | 1055 | 1055 |

Two further examples of tablets (10.3 and 10.4) were prepared by coating tablets 10.1 and 10.2 with Eudragit L.

### Example 11

Tablets 11.1 having a structure as shown in Fig. 1B were prepared

| | Amounts in mg |
|---|---|
| **First Layer** | **11.1** |
| AMX.3H₂O | 270.85 |
| (= AMX f.a.) | (234.0) |
| AMX.3H₂O/K-CA 1:1 | (206.90) |
| (= AMX f.a.) | (87.1) |
| (= CA f.a.) | (90.0) |
| Compritol 888 | 225.00 |
| Polyvidon K30 | 26.25 |
| Talc | 9.75 |
| Mg-Stearate | 11.25 |
| weight of first layer 1 | 750.00 |

| **Barrier layer** | S2 |
|---|---|
| **weight of layer 2** | 120 |

| **Second Layer** | |
|---|---|
| AMX.3H₂O | 167.86 |
| (= AMX f.a.) | (145.0) |
| AMX.3H₂O/K-CA 1:1 | 80.46 |
| (= AMX f.a.) | (33.9) |
| (= CA f.a.) | (35.0) |
| Avicel PH 102 | 53.21 |
| Explotab | 14.19 |
| Talc | 14.19 |
| Polyvidon K-30 | 12.42 |
| Syloid 244 | 7.09 |
| Mg-Stearate | 5.32 |
| | |
| weight of layer 3 | 345.74 |
| | |
| Total tablet weight | 1224.74 |

Potassium clavulanate was embedded in Compritol 888 by repeated steps of wet granulation. The first layer was a slow-release layer, and the second layer was a rapid-release layer.

### In Vitro and In Vivo Test Results

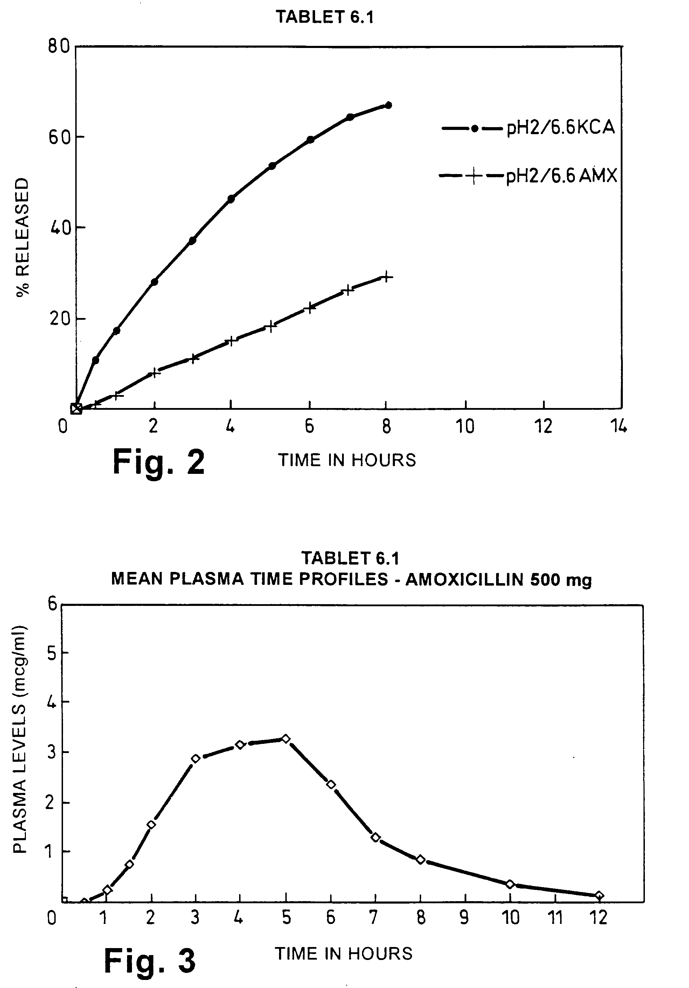

*In vitro* dissolution tests were carried out in a dual system, e.g. flowing media 2 hours at pH 2.2 followed by 6 hours at pH 6.6. Fig 2 shows results for tablet 6.1 from which amoxycillin and clavulanate were released with completion of release being almost reached within 8 hours.

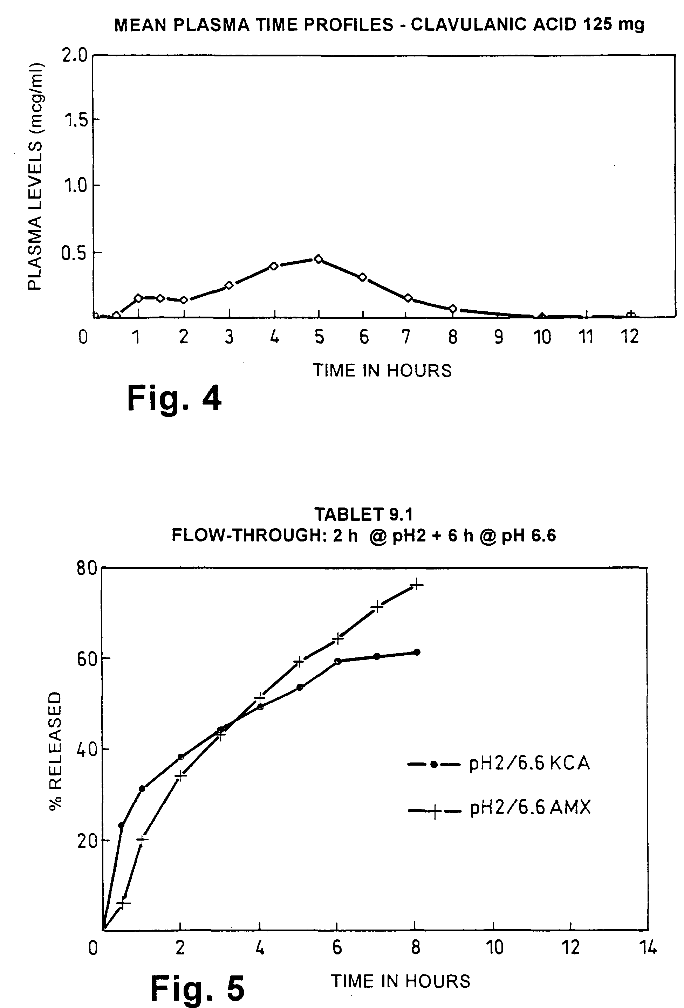

*In vivo* tests using tablet 6.1 were carried out on fed (milk) volunteers, and as shown in Fig 3 showed a prolonged amoxycillin plasma level. Similarly as shown in Fig 4 a prolonged release of clavulanate was also experienced. The tablet 6.1 consequently shows a prolonged release profile of amoxycillin and clavulanate.

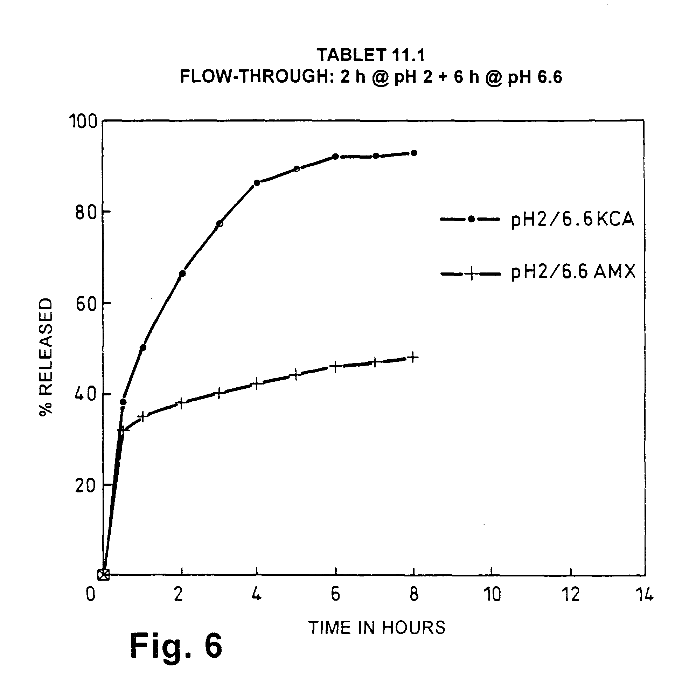

Fig.5 shows *in vitro* dissolution test results for tablet 9.1, and Fig.6 shows results for tablet 11.1, both showing prolongation of the release of clavulanate.

## Claims

1. A tablet formulation for oral administration, comprising a first layer which includes amoxycillin and/or clavulanate, and a second layer which includes amoxycillin and/or clavulanate, provided that the overall tablet contains amoxycillin and clavulanate, wherein the relative rate of release of amoxycillin and/or clavulanate from the first and second layers differs.

2. A tablet formulation according to claim 1 wherein the amoxycillin is in the form of amoxycillin trihydrate, and clavulanate is in the form of potassium clavulanate.

3. A tablet formulation according to claim 1 or 2 wherein:
(a) one layer contains amoxycillin without clavulanate and the other layer contains amoxycillin plus clavulanate;
(b) one layer contains clavulanate without amoxycillin and the other layer contains amoxycillin plus clavulanate; or
(c) one layer contains amoxycillin without clavulanate and the other layer contains clavulanate without amoxycillin;
making up the ratio in the overall tablet.

4. A tablet formulation according to any one of claims 1 to 3 wherein differing rates of release are achieved by a first layer which is a rapid release layer which releases the bulk of its active material content within a relatively short time, and a second layer which is a slow release layer which releases the bulk of its active material content during a relatively long period after oral ingestion or a delayed release layer which releases the bulk of its active material content after a period of delay after oral ingestion, either in the stomach or in the intestine.

5. A tablet formulation according to claim 4 wherein the rapid release layer is a rapid disintegrating layer.

6. A tablet formulation according to claim 4 wherein the rapid release layer is a swellable layer having a composition which incorporates polymeric materials which swell rapidly and extensively in contact with water or aqueous media, to form a water permeable but relatively large swollen mass.

7. A tablet formulation according to any one of claims 4 to 6 wherein the slow release layer comprises a release retarding material which is a pH sensitive polymer; a release-retarding polymer which has a high degree of swelling in contact with water or aqueous media; a polymeric material which forms a gel on contact with water or aqueous media, or a polymeric material which has both swelling and gelling characteristics in contact with water or aqueous media.

8. A tablet formulation according to any one of the preceding claims wherein differing rates of release are achieved by having a first layer which is a slow or delayed release layer, and a second layer which is also a slow or delayed release layer.

9. A tablet formulation according to claim 8 wherein one or both of the first or second layers is a delayed release layer using enteric polymers.

10. A tablet formulation according to any one of the preceding claims wherein the tablet includes one or more barrier layers.

11. A tablet formulation according to claim 10 wherein the barrier layer is located between the respective first and second layers, and/or on one or more of the outer surfaces of the first and second layers.

12. A tablet formulation according to claim 10 or 11 wherein the barrier layer(s) is/are composed of polymers which are either substantially or completely impermeable to water or aqueous media, or are slowly erodable in water or aqueous media or biological liquids and/or which swell in contact with water or aqueous media.

13. A tablet formulation according to any one of the preceding claims which comprises:
(a) a rapid disintegrating first layer which contains amoxycillin and/or clavulanate as active material, and a slow-release second layer which comprises amoxycillin and/or clavulanate as active material together with one or more release-retarding polymers, optionally with a barrier layer of either a completely impermeable polymer or a slowly erodable polymer sandwiched between the first and second layers, the overall tablet containing amoxycillin plus clavulanate;
(b) a slow-release first layer which contains amoxycillin and/or clavulanate as active material together with one or more release retarding polymers, and a second layer which is also a slow-release layer and which contains amoxycillin and/or clavulanate as active material together with one or more release retarding polymers, and a barrier layer of either a completely impermeable polymer or a slowly erodable polymer sandwiched between the first and second layers or located on one of the end faces of the tablet, the overall tablet containing amoxycillin plus clavulanate;
(c) a rapid release first layer which is a swellable layer containing amoxycillin, and a slow-release or delayed release second layer as described above containing amoxycillin, or clavulanate, or amoxycillin plus clavulanate, having a barrier layer of either a substantially or completely impermeable polymer or a slowly erodable polymer sandwiched between the first and second layers;
(d) a slow-release first layer comprising amoxycillin optionally together with clavulanate as active material, and a second layer which is a delayed release layer comprising a disintegrable or soluble matrix within which are dispersed enteric polymer coated granules which comprise clavulanate, optionally together with amoxycillin, with a barrier layer sandwiched between the first and second layers; or
(e) a first layer which is a slow release layer containing polymers which rapidly swell in contact with water or aqueous media so that the swollen mass is not rapidly discharged from the stomach containing amoxycillin or amoxycillin plus clavulanate as active material, and a second layer which is a slow release layer containing amoxycillin or amoxycillin plus clavulanate as active material, with a barrier layer of either a completely impermeable polymer or a slowly erodable polymer sandwiched between the first and second layers.

14. A tablet formulation according to any one of claims 1 to 13 which comprises a three-layer tablet in which all three of the layers include an active material content.

15. A tablet formulation according to claim 14 which comprises a slow-or delayed-release first layer which comprises amoxycillin, optionally together with clavulanate, a rapid-release second layer which comprises amoxycillin, optionally together with clavulanate, and a third layer, sandwiched between the first and second layers, and being a slow- or delayed-release layer comprising calvulanate, optionally together with amoxycillin.

16. A method for the manufacture of a tablet formulation according to any one of the preceding claims comprising the steps of forming said first and second layers, and any barrier layers and coating layer(s) which may be present.

17. A tablet formulation according to any one of claims 1 to 15 for use as a therapeutic substance for oral administration for the treatment of bacterial infections.

## Patentansprüche

1. Tablettenformulierung zur oralen Verabreichung, umfassend eine erste Schicht, die Amoxycilin und/oder Clavulanat enthält, und eine zweite Schicht, die Amoxycilin und/oder Clavulanat enthält, vorausgesetzt, dass die Gesamttablette Amoxycilin und Clavulanat enthält, wobei sich die relative Freisetzungsgeschwindigkeit von Amoxycilin und/oder Clavulanat in der ersten und zweiten Schicht unterscheidet.

2. Tablettenformulieruag nach Anspruch 1, wobei Amoxycilin in Form von Amoxycilin-Trihydrat und Clavulanat in Form von Kaliumclavulanat vorliegt.

3. Tablettenformulierung nach Anspruch 1 oder 2, wobei:
(a) eine Schicht Amoxycilin ohne Clavulanat enthält und die andere Schicht Amoxycilin und Clavulanat enthält;
(b) eine Schicht Clavulanat ohne Amoxycilin enthält und die andere Schicht Amoxycilin und Clavulanat enthält; oder
(c) eine Schicht Amoxycilin ohne Clavulanat enthält und die andere Schicht Clavulanat ohne Amoxycilin enthält;
und diese das Verhältnis in der Gesamttablette ausmachen.

4. Tablettenformulierung nach einem der Ansprüche 1 bis 3, wobei unterschiedliche Freisetzungsgeschwindigkeiten durch eine erste Schicht, welche eine schnell freisetzende Schicht ist, die den Großteil ihres Wirkstoffgehaltes innerhalb relativ kurzer Zeit freisetzt, und eine zweite Schicht, die eine langsam freisetzende Schicht ist, die den Großteil ihres Wirkstoffgehaltes während eines relativ langen Zeitraums nach der oralen Einnahme freisetzt, oder eine verzögert freisetzende Schicht ist, die den Großteil ihres Wirkstoffgehaltes nach der oralen Einnahme nach einer zeitlichen Verzögerung entweder im Magen oder im Darm freisetzt, erzielt werden.

5. Tablettenformulierung nach Anspruch 4, wobei die schnell freisetzende Schicht eine schnell zerfallende Schicht ist.

6. Tablettenformulierung nach Anspruch 4, wobei die schnell freisetzende Schicht eine quellfähige Schicht ist, deren Zusammensetzung Polymermaterialien enthält, die bei Kontakt mit Wasser oder wässrigen Medien rasch quellen, so dass eine wasserdurchlässige, jedoch relativ große gequollene Masse entsteht.

7. Tablettenformulierung nach einem der Ansprüche 4 bis 6, wobei die langsam freisetzende Schicht ein freisetzungsverzögerndes Material, welches ein pH-Wert-empfindliches Polymer ist; ein freisetzungsverzögerndes Polymer, das bei Kontakt mit Wasser oder wässrigen Medien hochgradig quillt; ein Polymermaterial, das bei Kontakt mit Wasser oder wässrigen Medien ein Gel bildet; oder ein Polymermaterial, das bei Kontakt mit Wasser oder wässrigen Medien sowohl Quellungs- als auch Gelbildungseigenschaften aufweist, umfasst.

8. Tablettenformulierung nach einem der vorhergehenden Ansprüche, wobei unterschiedliche Freisetzungsgeschwindigkeiten mit einer ersten Schicht, die eine langsam oder verzögert freisetzende Schicht ist, und einer zweiten Schicht, die ebenfalls eine langsam oder verzögert freisetzende Schicht ist, erzielt werden.

9. Tablettenformulierung nach Anspruch 8, wobei eine oder beide der ersten oder zweiten Schichten eine verzögert freisetzende Schicht ist, die darmlösliche Polymere verwendet.

10. Tablettenformulierungen nach einem der vorhergehenden Ansprüche, wobei die Tablette eine oder mehrere Sperrschichten umfasst.

11. Tablettenformulierung nach Anspruch 10, wobei sich die Sperrschicht zwischen der jeweils ersten und zweiten Schicht befindet, und/oder auf einer oder mehreren Außenflächen der ersten und zweiten Schicht.

12. Tablettenformulierung nach Anspruch 10 oder 11, wobei die Sperrschicht(en) aus Polymeren besteht/bestehen, die entweder im Wesentlichen oder vollständig gegenüber Wasser oder wässrigen Medien undurchlässig sind, oder die sich in Wasser oder wässrigen Medien oder biologischen Flüssigkeiten langsam zersetzen, und/oder die bei Kontakt mit Wasser oder wässrigen Medien quellen.

13. Tablettenformulierung nach einem der vorhergehenden Ansprüche, umfassend:
(a) eine sich rasch zersetzende erste Schicht, die Amoxycilin und/oder Clavulanat als Wirkstoff enthält, und eine langsam freisetzende zweite Schicht, umfassend Amoxycilin und/oder Clavulanat als Wirkstoff zusammen mit einem oder mehreren abgabeverzögernden Polymeren, gegebenenfalls mit einer Sperrschicht aus einem vollständig undurchlässigen Polymer oder einem sich langsam zersetzenden Polymer, welche sich zwischen der ersten und zweiten Schicht befindet, wobei die Gesamttablette Amoxycilin und Clavulanat enthält;
(b) eine langsam freisetzende erste Schicht, die Amoxycilin und/oder Clavulanat als Wirkstoff zusammen mit einem oder mehreren freisetzungsverzögernden Polymeren enthält, und eine zweite Schicht, die ebenfalls eine langsam freisetzende Schicht ist und Amoxycilin und/oder Clavulanat als Wirkstoff und ein oder mehrere freisetzungsverzögernde Polymere enthält, sowie eine Sperrschicht aus entweder einem vollständig undurchlässigen Polymer oder einem sich langsam zersetzbaren Polymer, welche sich zwischen der ersten und zweiten Schicht befindet oder sich an einer der Endflächen der Tablette befindet, wobei die Gesamttablette Amoxycilin und Clavulanat enthält;
(c) eine schnell freisetzende erste Schicht, welche eine quellfähige Schicht ist und Amoxycilin enthält, und eine langsam freisetzende oder verzögert freisetzende zweite Schicht wie vorstehend beschrieben, die Amoxycilin oder Clavulanat oder Amoxycilin und Clavulanat enthält, mit einer Sperrschicht aus entweder einem im Wesentlichen oder vollständig undurchlässigen Polymer oder einem sich langsam zersetzenden Polymer, welche sich zwischen der ersten und zweiten Schicht befindet;
(d) eine langsam freisetzende erste Schicht, die Amoxycilin gegebenenfalls zusammen mit Clavulanat als Wirkstoff umfasst, und eine zweite Schicht, die eine verzögernd freisetzende Schicht ist und eine zersetzbare oder lösliche Matrix umfasst, in der mit einem darmlöslichen Polymer beschichtete Granula dispergiert sind, welche Clavulanat gegebenenfalls zusammen mit Amoxycilin umfassen, mit einer Sperrschicht, die sich zwischen der ersten und zweiten Schicht befindet; oder
(e) eine erste Schicht, die eine langsam freisetzende Schicht ist und Polymere enthält, die bei Kontakt mit Wasser oder wässrigen Medien rasch quellen, so dass die gequollene Masse nicht schnell aus dem Magen ausgeschieden wird, und Amoxycilin oder Amoxycilin und Clavulanat als Wirkstoff enthält, und eine zweite Schicht, die eine langsam freisetzende Schicht ist und Amoxycilin oder Amoxycilin und Clavulanat als Wirkstoff enthält, mit einer Sperrschicht aus entweder einem vollständig undurchlässigen Polymer oder einem sich langsam zersetzenden Polymer, welche sich zwischen der ersten und zweiten Schicht befindet.

14. Tablettenformulierung nach einem der Ansprüche 1 bis 13, umfassend eine dreischichtige Tablette, bei der alle drei Schichten Wirkstoff enthalten.

15. Tablettenformulierung nach Anspruch 14, umfassend eine langsam oder verzögert freisetzende erste Schicht, die Amoxycilin, gegebenenfalls zusammen mit Clavulanat umfasst, eine schnell freisetzende zweite Schicht, die Amoxycilin gegebenenfalls zusammen mit Clavulanat umfasst, und eine dritte Schicht, die sich zwischen der ersten und zweiten Schicht befindet und eine langsam oder verzögert freisetzende Schicht ist und Clavulanat gegebenenfalls zusammen mit Amoxycilin umfasst.

16. Verfahren zur Herstellung einer Tablettenformulierung nach einem der vorhergehenden Ansprüche, umfassend die Schritte Herstellen der ersten und zweiten Schicht und jeglicher Sperrschichten und Überzugsschicht(en), die zugegen sein können.

17. Tablettenformulierung nach einem der Ansprüche 1 bis 15 zur Verwendung als Therapeutikum zur oralen Verabreichung für die Behandlung bakterieller Infektionen.

## Revendications

1. Formulation de comprimé pour l'administration orale, comprenant une première couche qui comprend de l'amoxycilline et/ou du clavulanate, et une seconde couche qui comprend de l'amoxycilline et/ou du clavulanate, sous réserve que le comprimé total contienne de l'amoxycilline et du clavulanate, dans laquelle les vitesses relatives de libération d'amoxycilline et/ou de clavulanate par les première et seconde couches diffèrent.

2. Formulation de comprimé suivant la revendication 1, dans laquelle l'amoxycilline est sous forme de trihydrate d'amoxycilline, et le clavulanate est sous forme de clavulanate de potassium.

3. Formulation de comprimé suivant la revendication 1 ou 2, dans laquelle :
(a) une couche contient de l'amoxycilline sans clavulanate et l'autre couche contient de l'amoxycilline plus du clavulanate ;
(b) une couche contient du clavulanate sans amoxycilline et l'autre couche contient de l'amoxycilline plus du clavulanate ; ou
(c) une couche contient de l'amoxycilline sans clavulanate et l'autre couche contient du clavulanate sans amoxycilline ;
en le rapport donnant le comprimé total.

4. Formulation de comprimé suivant l'une quelconque des revendications 1 à 3, dans laquelle des vitesses différentes de libération sont atteintes par une première couche qui est une couche à libération rapide qui libère la plus grande partie de sa quantité de substance active en un temps relativement bref, et une seconde couche qui est une couche à libération lente qui libère la plus grande partie de sa quantité de substance active en un temps relativement long après l'ingestion orale ou une couche à libération retardée qui libère la plus grande partie de sa quantité de substance active après une période de retard après l'ingestion orale, dans l'estomac ou bien dans l'intestin.

5. Formulation de comprimé suivant la revendication 4, dans laquelle la couche à libération rapide est une couche à délitement rapide.

6. Formulation de comprimé suivant la revendication 4, dans laquelle la couche à libération rapide est une couche pouvant gonfler, ayant une composition qui renferme la matière polymère qui gonfle rapidement et fortement au contact de l'eau ou d'un milieu aqueux, pour former une masse gonflée perméable à l'eau mais relativement volumineuse.

7. Formulation de comprimé suivant l'une quelconque des revendications 4 à 6, dans laquelle la couche à libération lente comprend une substance retardant la libération qui est un polymère sensible au pH ; un polymère retardant la libération qui a un haut degré de gonflement au contact de l'eau ou d'un milieu aqueux ; une matière polymère qui forme un gel au contact de l'eau ou d'un milieu aqueux, ou une matière polymère qui présente à la fois des caractéristiques de gonflement et des caractéristiques de gélification au contact de l'eau ou d'un milieu aqueux.

8. Formulation de comprimé suivant l'une quelconque des revendications précédentes, dans laquelle des vitesses différentes de libération sont atteintes par la présence d'une première couche qui est une couche à libération lente ou retardée, et d'une seconde couche qui est également une couche à libération lente ou retardée.

9. Formulation de comprimé suivant la revendication 8, dans laquelle l'une des, ou les deux, première et seconde couches consistent en une couche à libération retardée utilisant des polymères entériques.

10. Formulation de comprimé suivant l'une quelconque des revendications précédentes, dans laquelle le comprimé comprend une ou plusieurs couches d'arrêt.

11. Formulation de comprimé suivant la revendication 10, dans laquelle la couche d'arrêt est située entre les première et seconde couches respectives, et/ou sur une ou plusieurs des surfaces extérieures des première et seconde couches.

12. Formulation de comprimé suivant la revendication 10 ou 11, dans laquelle la ou les couches d'arrêt sont constituées de polymères qui sont substantiellement ou totalement imperméables à l'eau ou à un milieu aqueux, ou qui sont lentement érodables dans l'eau ou un milieu aqueux ou des liquides biologiques et/ou qui gonflent au contact de l'eau ou d'un milieu aqueux.

13. Formulation de comprimé suivant l'une quelconque des revendications précédentes, qui comprend :
(a) une première couche à délitement rapide qui contient de l'amoxycilline et/ou du clavulanate comme substance active, et une seconde couche à libération lente qui comprend de l'amoxycilline et/ou du clavulanate comme substance active conjointement avec un ou plusieurs polymères retardant la libération, facultativement avec une couche d'arrêt constituée d'un polymère totalement imperméable ou d'un polymère lentement érodable intercalée entre les première et seconde couches, le comprimé total contenant de l'amoxycilline plus du clavulanate ;
(b) une première couche à libération lente qui contient de l'amoxycilline et/ou du clavulanate comme substance active conjointement avec un ou plusieurs polymères retardant la libération, et une seconde couche qui est également une couche à libération lente et qui contient de l'amoxycilline et/ou du clavulanate comme substance active conjointement avec un ou plusieurs polymères retardant la libération, et une couche d'arrêt constituée d'un polymère totalement imperméable ou d'un polymère lentement érodable intercalée entre les première et seconde couches ou située sur une des faces terminales du comprimé, le comprimé total contenant de l'amoxycilline plus du clavulanate ;
(c) une première couche à libération rapide qui est une couche pouvant gonfler contenant de l'amoxycilline, et une seconde couche à libération lente ou à libération retardée répondant à la description précitée contenant de l'amoxycilline, ou du clavulanate, ou de l'amoxycilline plus du clavulanate, comportant une couche d'arrêt constituée d'un polymère substantiellement ou totalement imperméable ou d'un polymère lentement érodable intercalée entre les première et seconde couches ;
(d) une première couche à libération lente comprenant de l'amoxycilline facultativement en association avec du clavulanate comme substance active, et une seconde couche qui est une couche à libération retardée comprenant une matrice apte au délitement ou soluble dans laquelle sont dispersés des granules enrobés d'un polymère entérique, qui comprennent du clavulanate, facultativement en association avec de l'amoxycilline, avec une couche d'arrêt intercalée entre les première et seconde couches ; ou
(e) une première couche qui est une couche à libération lente contenant des polymères qui gonflent rapidement au contact de l'eau ou d'un milieu aqueux, de telle sorte que la masse gonflée ne soit pas déchargée rapidement de l'estomac, contenant de l'amoxycilline ou de l'amoxycilline plus du clavulanate comme substance active, et une seconde couche qui est une couche à libération lente contenant de l'amoxycilline ou de l'amoxycilline plus du clavulanate comme substance active, avec une couche d'arrêt constituée d'un polymère totalement imperméable ou d'un polymère lentement érodable intercalée entre les première et seconde couches.

14. Formulation de comprimé suivant l'une quelconque des revendications 1 à 13, qui comprend un comprimé en trois couches dans lequel l'ensemble des trois couches comprend une quantité de substance active.

15. Formulation de comprimé suivant la revendication 14, qui comprend une première couche à libération lente ou retardée qui comprend de l'amoxycilline, facultativement en association avec du clavulanate, une seconde couche à libération rapide qui comprend de l'amoxycilline, facultativement en association avec du clavulanate, et une troisième couche, intercalée entre les première et seconde couches et consistant en une couche à libération lente ou retardée comprenant du clavulanate, facultativement en association avec de l'amoxycilline.

16. Procédé pour la production d'une formulation de comprimé suivant l'une quelconque des revendications précédentes, comprenant les étapes consistant à former lesdites première et seconde couches, et n'importe quelles couches d'arrêt et une ou plusieurs couches d'enrobage quelconques qui peuvent être présentes.

17. Formulation de comprimé suivant l'une quelconque des revendications 1 à 15, destinée à être utilisée comme substance thérapeutique pour l'administration orale à des fins de traitement d'infections bactériennes.
